Europäisches Patentamt

European Patent Office

Office européen des brevets

(19)

(11) Veröffentlichungsnummer: **0 265 662**
**A2**

(12) # EUROPÄISCHE PATENTANMELDUNG

(21) Anmeldenummer: **87113784.0**

(51) Int. Cl.⁴: **A61K 35/78**

(22) Anmeldetag: **21.09.87**

(30) Priorität: **22.09.86 DE 3632173**

(43) Veröffentlichungstag der Anmeldung:
**04.05.88 Patentblatt 88/18**

(84) Benannte Vertragsstaaten:
**AT BE CH DE ES FR GB IT LI NL SE**

(71) Anmelder: **Badmajew, Vladimir, Dr.**
**1440/6 Forest Hill Road**
**Staten Island New York 10314(US)**

(72) Erfinder: **Badmajew, Vladimir, Dr.**
**1440/6 Forest Hill Road**
**Staten Island New York 10314(US)**

(74) Vertreter: **Bauer, Wulf, Dr.**
**Wolfgang-Müller-Strasse 12**
**D-5000 Köln 51 (Marienburg)(DE)**

(54) **Pharmazeutikum.**

(57) Das Pharmazeutikum ist vorwiegend und vorzugsweise ausschließlich aus zerkleinerten, pflanzlichen Bestandteilen zusammengesetzt, in Kombination weist es folgende Bestandteile auf:
Achaciae catechu flores aus der Familie der Leguminosae, Andropogon muriaticus aus der Familie der Graminae und Holz des Santalum album aus der Familie der Santalaceae.

EP 0 265 662 A2

## Pharmazeutikum

Die Erfindung bezieht sich auf ein Pharmazeutikum nach dem Oberbegriff des Anspruchs 1 und dem Oberbegriff des Anspruchs 8, das vorwiegend, vorzugsweise ausschließlich aus zerkleinerten, pflanzlichen Bestandteilen zusammengepreßt ist

Das erfindungsgemäße Pharmazeutikum setzt sich aus folgenden Bestandteilen in Kombination zusammen: Acaiae catechu flores aus der Familie der Leguminosae, Andropogon muriaticus aus der Familie der Graminae und aus Holz des Santalum album aus der Familie der Santalaceae. In einer Weiterbildung sind die Bestandteile Acaciae catechu flores (kurz: ACF), Andropogon muriaticus (kurz: AM) und Santalum album (kurz: SA) im Gewichtsverhältnis 5:23:13 vorgesehen. In einer anderen Weiterbildung wird vorgeschlagen, daß als zusätzlicher Bestandteil Corallum und/oder Quisqualis sinensis (kurz: QQS) und/oder Selaginella involvens (kurz: SI) und/oder Hemidesmi indici radices aus der Familie der Asclepiadaceae enthalten ist.

Dieses Pharmazeutikum ist in erster Linie für die Behandlung von Streßauswirkungen angezeigt. Diese therapeutische Wirkung wird auf die Zusammenwirkung der drei wesentlichen Bestandteile, nämlich ACF, AM und SA zurückgeführt. Diese Bestandteile sind in ihrer Kombination, insbesondere in ihrer gewichtsmäßigen Abstufung innerhalb der Kombination, neu.

Weiterhin ist das Pharmazeutikum wirksam gegen kardiovaskuläre, immunologische und neurologische Krankheiten. Am Menschen und am Tier untersucht, wird auch hier die Heilwirkung auf den Abbau negativer Streßauswirkungen zurückgeführt.

Aufgrund der entscheidenden Bestandteile ACF, AM und SA in Kombination wird die Sekretion von Neurohormonen, beispielsweise Catecholaminen, stabilisiert, die für eine Erhöhung von Herzfrequenz und Vasospasmus verantwortlich sind. Das Pharmazeutikum hat sich insbesondere bei der Behandlung der Auswirkungen von chronischem Streß bewährt, wie er bei Individuen auftritt, die dem Typ A zugeordnet werden. Derartige A-Individuen haben ein Verhalten, das charakterisiert ist durch starken Antrieb, Wettbewerbsbewußtsein, ständiges Gefühl der Zeitknappheit, Hast und Gegnerschaft. Durch die die Streßauswirkung abbauenden drei entscheidenden Bestandteile ACF, HIR und SA werden diese Charakteristika reduziert und die Fähigkeit zur Konzentration und zu effektiver Arbeit erhöht. Dieses therapeutische Ziel kann durch eine präventive Dosis von einer Tablette zu 0,5 g pro Tag erreicht werden. Nebenwirkungen wurden bei einer derartigen Behandlung nicht beobachtet.

Die streßabbauenden Eigenschaften des Pharmazeutikums lassen es angezeigt erscheinen, dieses Pharmazeutikum bei der Behandlung von Krankheiten einzusetzen, bei denen Streßfaktoren eine Rolle spielen.

So finden sich beispielsweise in der Literatur Hinweise für einen Zusammenhang zwischen Streß und Erkrankungen der Herzkranzgefäße. Nach einer Schätzung trägt Streß zwischen 36 bis 70 Prozent zur Entstehung von Bluthochdruck bei. Es gibt auch zunehmende Hinweise dafür, daß akuter oder chronischer Streß eine Vielzahl von Krankheiten auslöst, wie beispielsweise geschwächte Abwehr gegen Infektionen durch Viren oder Mikroben.

In einer bevorzugten Ausführung sind im Pharmazeutikum enthalten:

ACF        0,05 g
AM = Andropogon muriaticus (Leguminosae)        0,23 g
SA        0,13 g.

Im folgenden werden zwei Ausführungsbeispiele angegeben.

Der therapeutische Effekt beruht auf einem Ergebnis der Wechselwirkung zwischen ACF, AM, SA und den anderen Bestandteilen. Das Pharmazeutikum weist pflanzliche Bestandteile auf, die hypotensiv sind, beispielsweise Valerianae radix, ACF, die zusammen mit harntreibenden Bestandteilen wie beispielsweise SA die Kreislaufbelastung abbauen, wodurch die Blutzirkulation verbessert wird. Das Pharmazeutikum enthält auch Pflanzenbestandteile, die das emotionale Verhalten stabilisieren, beispielsweise Lactucae satival und AM. Neue Untersuchungen zeigen, daß emotionale Spannung die Blutzirkulation negativ beeinflussen kann. Zusätzlich hat das Pharmazeutikum Pflanzenbestandteile mit entzündungshemmender und schmerzlindernder Wirkung, wie beispielsweise Hedychii spicati und SA.

| Botanischer Name | Dosis in g | |
| --- | --- | --- |
| | Beispiel I | Beispiel II |
| Aegle sepiariae fructus | 0,23 | 0,02 |
| Acaciae catechu flores | 0,05 | 0,05 |
| Aconiti tuber | 0,01 | 0,01 |
| Amomi medicinalis fructus | 0,17 | 0,025 |
| Andropogon muriaticus | 0,23 | 0,23 |
| Aquilegia viridiae foliae | 0,11 | 0,015 |
| Calcium sulphate | 0,20 | 0,02 |
| Calendulae flores | 0,10 | 0,005 |
| Camphora japonicum | 0,06 | 0,02 |
| Cardamomi fructus | 0,19 | 0,03 |
| Caryophylli fructus | 0,08 | 0,012 |
| Costus amari radix | 0,18 | 0,04 |
| Corallum | 0,17 | 0 |
| Hedychii spicati rhizoma | 0,09 | 0,01 |
| Hemidesmi indici radix | 0,10 | 0,1 |
| Lactucae satial foliae satival | 0,08 | 0,006 |
| Lichen islandicus | 0,48 | 0,04 |
| Liquiritiae radix | 0,18 | 0,015 |
| Melia toosendan fructus | 0,14 | 0,035 |
| Myrobalan | 0,28 | 0,030 |
| Plantaginis lancefoliae herba | 0,10 | 0,015 |
| Polygoni avicularis herba | 0,20 | 0,015 |
| Potentillae aureae herba | 0,10 | 0,015 |
| Quisqualis sinensis | 0,23 | 0 |
| Santalini rubrum lignum | 0,26 | 0,03 |
| Santalum album | 0,13 | 0,13 |
| Selaginella involvens | 0,18 | 0 |
| Sidae cordifoliae radix | 0,10 | 0,01 |
| Valerianae radix | 0,16 | 0,01 |

Weiterhin bezieht sich die Erfindung auf ein Pharmazeutikum, das ebenfalls zur Therapie von Streßzuständen eingesetzt werden kann und das aus den folgenden pflanzlichen Bestandteilen, die jeweils zerkleinert sind, zusammengesetzt ist: Carthami tinctorii flores (kurz: CTF), Guaiacum officinale (kurz: GO), Pardanthi dichotomi radices (kurz: PDR), Parmeliae herba (kurz: PH) und Samen von Pyrus baecata (kurz: PB). In einer bevorzugten Ausführung ist das Gewichtsverhältnis dieser entscheidenden, in Kombination wirkenden fünf Bestandteile CTF, GO, PDR, PH und Samen von PB 9:10:23:48:17. Klinische Anwendung findet dieses Pharmazeutikum gegen Herzgefäßkrankheiten, beispielsweise Krankheiten der Herzkranzgefäße, Angina pectoris, Herzaussetzen durch Blutandrang. Daneben ist eine Therapie in der Dermatologie, z. B. bei Psoriasis und allergische Dermatitis gegeben. Eine weitere Indikation findet das Pharmazeutikum in der Immunologie, beispielsweise bei durch Abwehrschwäche hervorgerufenen chronischen Infektionen, bei Bronchialasthma sowie bei chronischer und aggressiver Hepatitis.

In einer 500 mg-Tablett sind von den entscheidenden Wirkstoffen enthalten:

CTF  9 mg
GO  10 mg
PDR  23 mg
PH  48 mg
Samen von
PB  17 mg.

Zu diesen Bestandteilen können die obengenannten Bestandteile hinzugefügt werden.

Die Verabreichung der beschriebenen Pharmazeutika erfolgt in homöopathischen Dosen, wobei eine homöopathische Dosierung bei den ausschließlich pflanzlichen Bestandteilen schon dadurch gegeben ist, daß in diesen die eigentlichen Wirkstoffe nur in sehr geringen Mengen vorhanden sind, und der größte Anteil der als Wirkbestandteile beschriebenen pflanzlichen Bestandteile Ballaststoffe sind. Diese Ballaststoffe haben wiederum einen positiven Effekt auf den Verdauungstrakt, wodurch dieser ordnungsgemäß funktioniert und hierdurch die Voraussetzungen für eine gute Aufnahme der Wirkstofe gegeben sind.

Die Pharmazeutika bestehen aus einer Mischung fein zerkleinerter Bestandteile, die zu Tabletten verpreßt wurde. Die sich leicht auflösenden Tabletten haben vorzugsweise keinen Überzug, dadurch wird ihr leicht bitterer Geschmack im Mund wahrgenommen, was zu einer Anregung der Speichelsekretion und somit zu besserer Resorption führt.

Die beschriebenen Pharmazeutika werden oral eingenommen.

**Ansprüche**

1. Pharmazeutikum, das vorwiegend, vorzugsweise ausschließlich aus zerkleinerten, pflanzlichen Bestandteilen zusammengesetzt ist, dadurch gekennzeichnet, daß es folgende Bestandteile in Kombination aufweist:
Achaciae catechu flores aus der Familie der Leguminosae, Andropogon muriaticus aus der Familie der Graminae und Holz des Santalum album aus der Familie der Santalaceae.

2. Pharmazeutikum nach Anspruch 1, dadurch gekennzeichnet, daß es die Bestandteile Acaciae catechu flores, Andropogon muriaticus und Santalum album im Gewichtsverhältnis 5:23:13 enthält.

3. Pharmazeutikum nach Anspruch 1 oder 2, dadurch gekennzeichnet, daß es als zusätzlichen Bestandteil Hemidesmi indici radices aus der Familie der Asclepiadaceae Corallum aufweist.

4. Pharmazeutikum nach einem der Ansprüche 1 bis 3, dadurch gekennzeichnet, daß es als zusätzlichen Bestandteil Quisqualis sinensis aufweist.

5. Pharmazeutikum nach einem der Ansprüche 1 bis 4, dadurch gekennzeichnet, daß es als zusätzlichen Bestandteil Selaginella involvens aufweist.

6. Pharmazeutikum nach einem der Ansprüche 1 bis 5, dadurch gekennzeichnet, daß die Bestandteile Acaciae catechu flores, Hemidesmi indici radices, Santalum album, Corallum, Quisqualis sinensis und Selaginella involvens im Gewichtsverhältnis 5:10:13:17:23:13 enthält

7. Verwendung des Pharmazeutikums nach einem der Ansprüche 1 bis 6 zur Behandlung von Streßzuständen.

8. Pharmazeutikum, das vorwiegend, vorzugsweise ausschließlich aus zerkleinerten, pflanzlichen Bestandteilen zusammengesetzt ist, dadurch gekennzeichnet, daß es folgende Bestandteile in Kombination aufweist: Carthami tinctorii flores aus der Familie der Compositae, Kernholz des Guaiacum officinale aus der Familie der Zygophyllaceae, Pardanthi dichotomi radices aus der Familie der Irideae, Parmeliae herba aus der Familie der Lichenes und Samen des Pyrus baecate aus der Familie der Rosaceae.

9. Pharmazeutikum nach Anspruch 8, dadurch gekennzeichnet, daß die Bestandteile Carthami tinctorii flores, Guaiacum officinale, Pardanthi dichotomi radices, parmeliae herba und Pyrus baecata im Gewichtsverhältnis 9:10:23:48:17 enthälten sind.

10. Verwendung eines Pharmazeutikums nach Anspruch 8 oder 9 zur Therapie von peripheren Gefäßkrankheiten, Angina pectoris, Herzaussetzen durch Blutandrang und Leberzirrhose.

11. Verwendung eines Pharmazeutikums nach den Ansprüchen 8 oder 9 zur Behandlung dermatologischer Krankheiten, beispielsweise Psoriasis und allergischer Dermatitis.

12. Verwendung eines Pharmazeutikums nach Anspruch 8 oder 9 in der Immunologie, insbesondere bei chronischen Infektionen und anderen Immunmangelkrankheiten. bei Bronchialasthma sowie bei aggressiver und chronischer Hepatitis.